Europäisches Patentamt

European Patent Office

Office européen des brevets

(11) Publication number: **0 346 091**
**A2**

# (12) EUROPEAN PATENT APPLICATION

(21) Application number: **89305735.6**

(22) Date of filing: **07.06.89**

(51) Int. Cl.⁴: **G 01 N 33/68**
// G01N33/569, C12N15/00

(30) Priority: **10.06.88 US 205168**

(43) Date of publication of application:
**13.12.89 Bulletin 89/50**

(84) Designated Contracting States:
**AT BE CH DE ES FR GB GR IT LI LU NL SE**

(71) Applicant: **DANA FARBER CANCER INSTITUTE**
**44 Binney Street**
**Boston Massachusetts 02115 (US)**

(72) Inventor: **Haseltine, William A.**
**24 Mt. Vernon Street**
**Cambridge Massachusetts 02140 (US)**

**Sodroski, Joseh Gerald**
**10, Ashland Place**
**Medford Massachusetts 02155 (US)**

**Kowalski, Mark**
**16, Circuit Rd.**
**Medford Massachusetts 02155 (US)**

**Ardman, Blair**
**395 Harvard St. No.3**
**Brookline Massachusetts 02146 (US)**

(74) Representative: **Davies, Jonathan Mark**
**Reddie & Grose 16 Theobalds Road**
**London WC1X 8PL (GB)**

(54) Method of diagnosis for naturally occurring antibodies to cd4.

(57) A method of diagnosis for naturally occurring antibodies against CD4 is disclosed.

Figure 1.

CMV·T4S

Description

# METHOD OF DIAGNOSIS FOR NATURALLY OCCURRING ANTIBODIES TO CD4

The present invention is directed to a method of diagnosis for naturally occurring antibodies in humans against CD4.

T-lymphocytes play an extremely important role in the immune response. Depletion of CD4+ lymphocytes can account for most of the severe immunologic abnormalities present in patients having the Acquired Immune Deficiency Syndrome (AIDS). This disease is characterized by a long asymptomatic followed by the progressive degeneration of the immune system and the central nervous systems. Studies of the virus indicate that replication is highly regulated and both latent and lytic infection of the CD4+ helper subset of T-lymphocytes occur in tissue culture [Zagury et al, Science 231:850-853 (1986)].

CD4+ lymphocyte depletion is seen in the majority of patients infected with the human immunodeficiency virus (HIV-I) prior to, or coincident with, the development of severe opportunistic infections. Furthermore, functional abnormalities have been reported to exist in CD4+ lymphocytes obtained from HIV-infected patients and such functional abnormalities may precede the loss of these cells in the course of the disease process. Accordingly, it is extremely important to identify mechanisms that can lead to CD4+ lymphocyte depletion and/or their functional defects. We have now discovered that certain individuals have naturally occurring anti-CD4 autoantibodies and disclose herewith a method of diagnosis for such naturally occurring antibodies in humans against CD4.

Naturally occurring antibodies in humans against CD4 can be determined by an assay which comprises adding a CD4 construct which contains a sufficient number of amino acids corresponding to an antigenic determinant of CD4 to a biological specimen and determining whether a reaction occurs with the CD4 construct. This CD4 construct can be selected from natural CD4, recombinant CD4, fragments or derivatives thereof. Preferably, a soluble CD4 product is used that does not contain the transmembrane sequence and the cytoplasmic region sequence of CD4. Preferably the biological specimen is human sera, although other body fluids from other species may be tested by the same procedure.

Figure 1 is a schematic of the soluble CD4-expressing plasmid, CMV-T4S.

Figure 2, A-C, are radiograms showing reaction of various sera with supernatant containing a control (2A) and recombinant soluble CD4 (2B and 2C).

Figure 3 are radiograms showing coprecipitation of soluble CD4 and HIV gp120 from COS-1 supernatants.

We have now discovered a method of diagnosis for the naturally occurring antibodies against CD4. This is preferably used for diagnosis of naturally occurring antibodies in humans.

It is disclosed herein that individuals possessing an autoimmune disease can naturally generate antibodies to the CD4 lymphocytes. These antibodies (sometimes referred to as anti-CD4 autoantibodies) might result in cytopathicity or immuno-unresponsiveness to a variety of pathogens, such as seen in individuals who suffer from AIDS. Thus, this test can be used to diagnose certain individuals having an autoimmune disease such as AIDS. It is expected that individuals with other autoimmune diseases, such as systemic lupus erythematosus and rheumatoid arthritis also produce these antibodies.

Although, it has been possible to generate antibodies to the CD4 glycoprotein in other species, for example, in mice, no naturally occurring antibodies to CD4 have previously been reported. As discussed above, we have found that in the course of HIV infection, some individuals can produce antibodies that bind to the virus receptor, the CD4 glycoprotein. It is also expected that this method can be used with other species infected with diseases that affect the immune system, e.g. SIV, to assay for naturally occurring antibodies to CD4.

Accordingly, based upon the present disclosure, it is possible to determine whether or not such antibodies are present in a person by using a CD4 construct that contains a sufficient number of amino acids corresponding to an antigenic determinant in CD4. For example, this CD4 construct can be either natural or recombinant CD4 fragments or derivatives thereof, including synthetic constructs. Any CD4 fragment which contains the proper antigenic determinant can be used. Preferably, one uses recombinant CD4 constructs as the antigen. More preferably, one uses soluble CD4, which does not contain the transmembrane sequence and cytoplasmic region sequence. [See, for example, Hussey, R.E., et al, Nature 331:78-81 (1988); Fisher, R.A., et al., Nature 331:76-78 (1988); Deen, K.C. et al, Nature 331:82-84 (1988) and Traunecker, A. et al, Nature 331:84-86 (1988), Smith D.H., et al, Science 238:1704-1707 (1987), all of which are incorporated herein by reference]. One does not need to use the entire soluble CD4, but merely a sufficient portion of the construct that contains antigenic determinants that react with the antibody. For example, some antigenic determinants that can react with the autoantibodies are predominantly located in the carboxy end of this soluble CD4 construct.

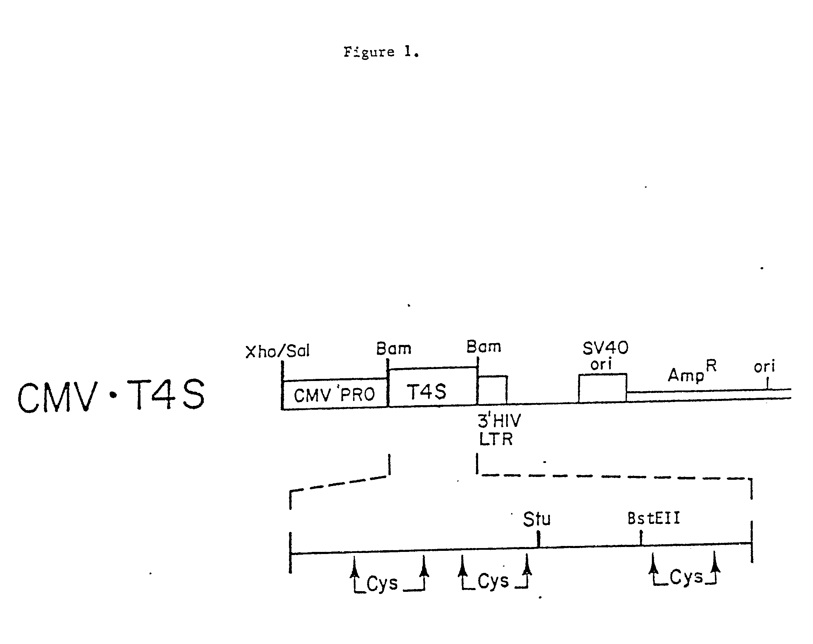

These constructs can be produced by a variety of means well known to the person of ordinary skill in the art. For example, they can be synthetically constructed, based upon the disclosed CD4 sequence, or expressed using the techniques disclosed in, for example, Hussey et al, Nature 331, supra. A CD4 expression seqeuence can be prepared by placing a stop codon just 5' to the nucleotide sequences encoding the transmembrane region of the wild-type CD4 protein, as disclosed in Hussey et al. This sequence can be inserted into an expression plasmid by standard techniques. In one preferred embodiment, these CD4 constructs are expressed in mammalian cells, for example, COS-1 (green monkey kidney) cells. A plasmid is selected containing a suitable promoter based upon the host cell to be used. For instance, in the soluble CD4 expressing plasmid CMV-T4S (See Figure 1) containing a cytomegalovirus early promoter (CMV), polyadenylation sequences and the SV40 origin, the CD4 sequence is inserted between the CMV promoter and the polyadenylation signals. Other expression plasmids, such as that shown in Deen, K.C., et al, Nature 331, supra, can also be used.

This plasmid is then used to transfect the desired mammalian cell, for example, COS-1 cells, by standard techniques. The soluble CD4 product is detectable in supernatant, rather than cell lysates and can be detected by means well known in the art, such as radioimmunoprecipitation.

The CD4 molecule can also be synthesized in bacteria, yeast, insect virus, or other expression systems for these purposes.

The presence of naturally occurring antibodies can be assayed by taking a predetermined biological sample, adding the CD4 construct to that sample and then determine if there is a reaction with the CD4 construct. For example, preparing CD4 from supernatants of metabolically labeled COS cells will result in labelled CD4. This labelled CD4 can be added directly to the biological sample, for example, patient sera, to be tested. Thereafter, the immunoprecipitates can be resolved by standard techniques, for example, SDS-PAGE, preferably, under non-reducing conditions, to determine if there is a reaction. We found that certain patients infected with HIV naturally produce an antibody that can radioimmunoprecipitate the soluble CD4 product.

Alternatively, one could use ELISA to determine, not only whether or not there is a reaction, but also to measure the titer of the reaction. With such a method, the soluble CD4 can be produced in a baculovirus expression system, such as disclosed in Hussey, et al, Nature 331, supra, for example, the $T4_{ext}$ expression system. Other prokaryotic or eukaryotic expression systems could be used to produce the CD4 product. The CD4-containing supernatant from the host cell can be purified by centrifugation, treated with protease inhibitors, and absorbed to an infinity matrix of OKT4A covalently linked to beads such as Affigel beads produced by BIORAD. In one instance, the bound material will be eluted under mild acidic conditions, neutralized, dialyzed and concentrated to form the CD4 surface. Thereafter, using standard techniques, the biological sample to be tested is added and a determination of binding and titer is made. With certain CD4 constructs containing conformation-dependent antigenic determinants, the ELISA tests may not be as preferable as, for example, a dot blotting assay. Beads containing the CD4 construct are another method. The particular assay that should be used for a particular CD4 construct can be readily determined by the person of ordinary skill in the art, based upon the present disclosure.

The presence or absence of antibodies can be used to determine the prognosis of the virus in the patient. For example, it is expected that there will be different antibodies to different antigenic determinants in the CD4. The determinants discussed above are located in the soluble portion. However, a construct containing determinants from the intracytoplasmic portion and not the soluble portion could be used to detect antibodies present in a different disease state. We believe that antibodies to the intracytoplasmic portion are not generated until a great deal of deterioration of the individual's CD4 cells has occurred. Determinants, based on the intracytoplasmic portion, can readily be prepared by modifying the technique discussed above with respect to preparing the soluble CD4 constructs e.g., by deleting the membrane-spanning anchorage region of the native CD4 molecule. Alternatively, peptides made in bacteria or other organisms or synthesized in vitro could be used as a source of the CD4 antigenic determinants recognized by human sera.

The present invention is further illustrated by the following examples. The examples are provided to aid in the understanding of the invention and are not to be construed as a limitation, thereof.

## Examples

COS-1 cells were transiently transfected with plasmid containing SV40 origins of replication to express readily detectable levels of HIV-1 gp120 and CD4 glycoproteins. The gp120 protein was expressed by transfecting the cell with an envelope expressing plasmid, pIIIenvΔ-517S, which is prepared by the method disclosed in Kowalski et al [Science 237:1351] (1987), which results in the secretion of a 517 amino acid product that contains six extra carboxy-terminal amino acids when compared to wild-type gp120.

The soluble CD4 was produced by using a CD4 expressing plasmid. Figure 1 shows the structure of this plasmid. The CMV-T4S plasmid expresses a soluble from of the human CD4 protein lacking its transmembrane region upon transient transfection into COS-1 cells. The cytomegalovirus early region promoter (CMV PRO) serves as the transcriptional promoter. The CMV immediate early promoter in this plasmid contains a Tha I fragment (-522 to +97 with respect to the cap site) from pCM5029 (Boshart et al., Cell 41: 521-530 (1985)). The soluble CD4 gene (T4S) contains an in-frame stop codon 5' to the sequences encoding the membrane-spanning region of the CD4 glycoprotein (Hussey et al., Nature, supra). The soluble CD4 protein made retains the ability to bind to the HIV gp120 envelope glycoprotein. Polyadenylation signals are provided by the 3' HIV long terminal repeat (LTR). The plasmid also contains an SV40 origin of replication (SV40 ori), a β-lactamase gene (Amp^R) and a bacterial origin of replication (ori).

In the lower portion of Figure 1, the position of codons for the cystein (cys) residues in the CD4 molecule are shown. The cysteines paired have been shown to be involved in intramolecular disulfide bonding. The position of the Stu I site is also shown.

After transient transfection by adding 5μg of each of the above-described plasmids to COS-cells, the majority of the soluble CD4 product was detectable by radioimmuno-precipitation of the supernatant, rather than cell lysates.

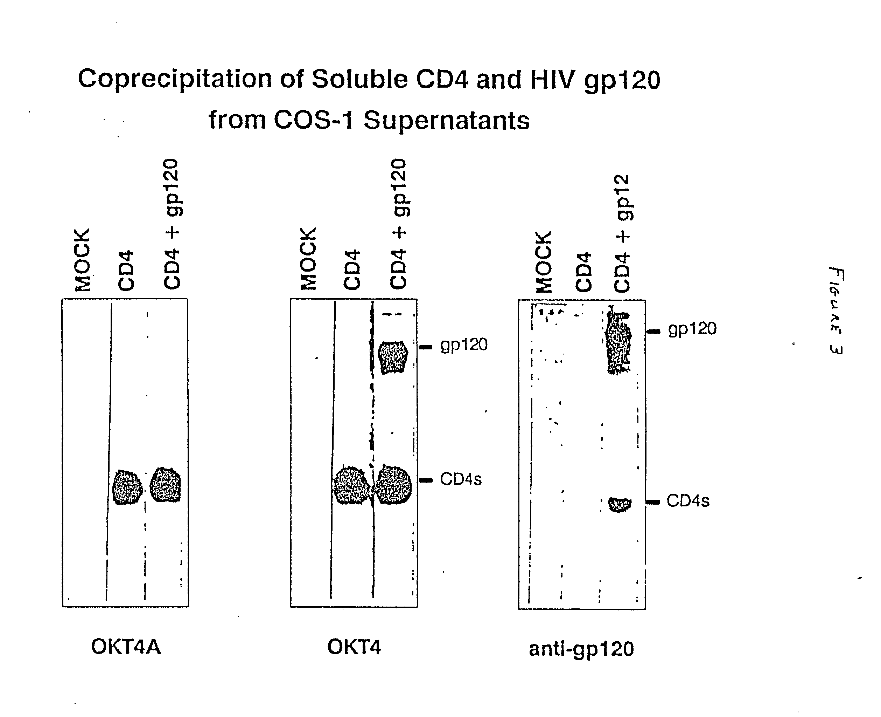

The cells were metabolically radiolabeled 48 hours post transfection and the supernatants were then immunoprecipitated with either OKT4A or OKT4. See Figure 3. In these co-transfected cells, both gp120 and the soluble CD4 product is co-precipitated by monoclonal antibodies directed against CD4 that do not block gp120-CD4 interaction. The OKT4 monoclonal antibody (Ortho Diagnostics) [McDougal, J., et al, Science 231:382 (1986) which is incorporated by reference] recognizes a region of CD4 distal from the gp120-binding region and will co-precipitate both CD4 and gp120 from COS-1 supernatants, although it does not precipitate gp120 when the envelope expressing plasmid is used alone for transfection. The OKT4A monoclonal antibody (Ortho Diagnostics) [McDougal, J., Science 231, supra] competes with gp120 for CD4 cells and only precipitated a soluble CD4 and not gp120 from supernatants of COS-1 cells that have been co-transfected as described above.

Thereafter, the CD4 expressing plasmid was used to transfect COS-1 cells by a modified calcium phosphate procedure [See Muesing, M., et al, Cell 48: 691 (1987) which is incorporated herein by reference. The COS-1 cell was metabolically labeled 48 hours post transfection with [$^{35}$S] methionine by standard techniques. Confluent 60mm plates were labeled at that time with 1.2 ml of methionine-free DME medium containing 1mCi of [$^{35}$S] methionine (1200 Ci/mmol) for 6 hours. The supernatant was collected, clarified by low speed centrifugation and diluted with an equal volume of 2 X RIPA buffer, cells were washed with phosphate-buffered saline and dissolved in 1 ml of 1 X RIPA buffer (1% Triton X-100, 0.5% sodium deoxycholate, 0.1% SDS, 0.12M NaCl, 0.05 M tris HCl, at pH 7.5). A predetermined amount of patient sera (5μl (?)] was added to the supernatant. Patient sera from 50 HIV-infected and 50 normal individuals were tested. The immunoprecipitates were resolved by SDS-PAGE under non-reducing conditions. The assay was incubated for hours at 4°C and immune complexes were collected with 10 l of Pansorbin. Precipitates were washed twice in 1 X RIPA and once in water, then eluted by heating at 100°C for 2 minutes in sample buffer (0.12M tris HCl, pH 6.8, 0.7M β-mercaptoethanol, 20% glycerol, 4% SDS and 0.1% bromophenol blue). Autoradiographs of the enhanced and dried cells were inspected for the presence of a 46 kD band that co-migrates with a positive control, anti-CD4 immunoprecipitate (OKT4A).

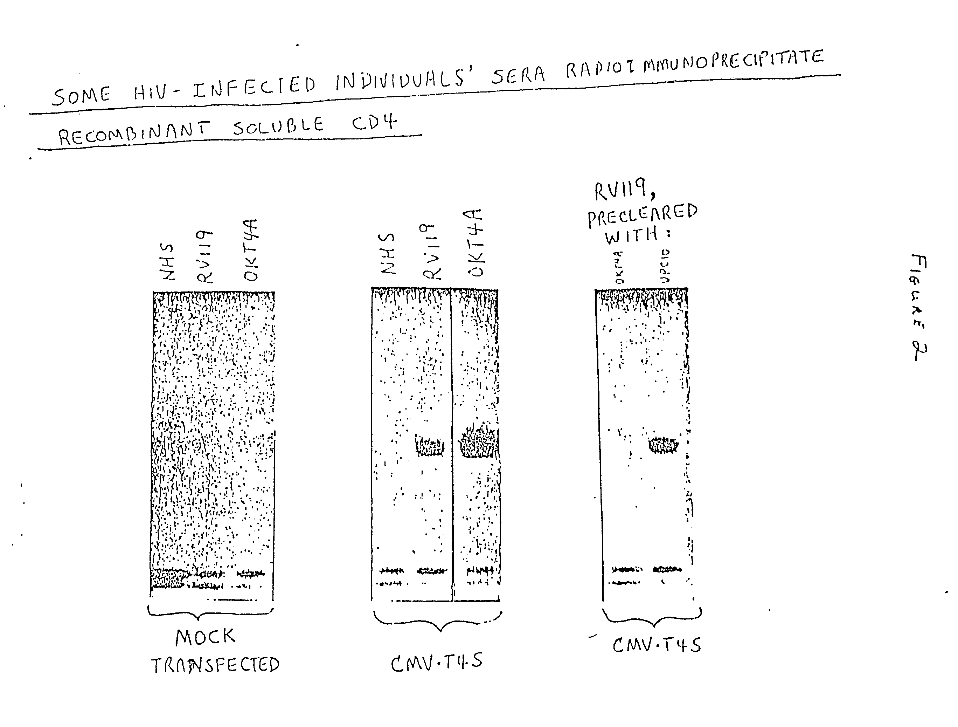

Supernatant from mock transfected COS-1 cells was also collected and assayed as described above. Figure 2A shows a autoradiograph from this mock-transfected cell. The first lane is uninfected human sera, the second lane is HIV-infected patient sera, the third lane adds the positive control (OKT4A) to the HIV-infected sera. Figure 2B uses supernatant from COS-1 cells transfected with the plasmid CMV4S (soluble CD4 expressing supernatant) with lanes as described above. Figure 2C uses the HIV-infected sera (RV119) from 2B to confirm that the positive control and the CD4 construct are reacting with the same protein. An excess of OKT4A is added to the sera in lane 1, precipitated out, than CD4 is added and assayed. In lane 2, an excess of UPC10 was added as a control and precipitated out before the CD4 was added. This shows that the RV119 serum is reacting with the same protein as the OKT4A.

No reactions to the CD4 were seen in the sera from any of fifty uninfected individuals tested, while a reaction in five of forty HIV infected individuals was observed.

It is evident that those skilled in the art, given the benefit of the foregoing disclosure, may make numerous modifications thereof and departures from the specific embodiments described herein without departing from the inventive concepts and the present invention is to be limited solely by the scope and spirit of the appended claims.

## Claims

1. A method of assay for naturally occurring antibodies against CD4, which comprises:
   (a) taking a predetermined sample;
   (b) adding a CD4 construct, which comprises a sufficient number of amino acids corresponding to CD4 to contain an antigenic determinant, to, the sample and
   (c) determining whether a complex is formed with the CD4 construct.

2. The method of claim 1, wherein the CD4 construct is selected from the group consisting of naturally occurring CD4, chemically synthesized CD4, recombinant CD4, fragments and derivatives thereof.

3. The method of claim 1, wherein the antibodies occur in humans.

4. The method of claim 2, wherein the CD4 construct is a recombinant soluble CD4 fragment.

5. The method of claim 1, wherein the determination is made by radioimmunoassay.

6. The method of claim 1, wherein the determination is made by ELISA.

7. The method of claim 1, wherein the determination is made by a dot-blot assay.

Figure 1.

EP 0 346 091 A2

SOME HIV-INFECTED INDIVIDUALS' SERA RADIOIMMUNOPRECIPITATE

RECOMBINANT SOLUBLE CD4

NHS
RVII9
OKT4A

NHS
RVII9
OKT4A

RVII9,
PRECLEARED
WITH:

OKT4A
UPC10

MOCK
TRANSFECTED

CMV·T4·S

CMV·T4S

FIGURE 2

FIGURE 3

Coprecipitation of Soluble CD4 and HIV gp120
from COS-1 Supernatants

OKT4A

MOCK
CD4
CD4 + gp120

OKT4

MOCK
CD4
CD4 + gp120
gp120
CD4s

anti-gp120

MOCK
CD4
CD4 + gp12
gp120
CD4s